# EUROPÄISCHE PATENTSCHRIFT

(11) **EP 2 440 147 B1**
(45) Veröffentlichungstag und Bekanntmachung des Hinweises auf die Patenterteilung: **14.08.2013**
(21) Anmeldenummer: 10737747.5
(22) Anmeldetag: 08.06.2010
(51) Int. Cl.: A61B 17/86, A61B 17/88

(54) **CHIRURGIEINSTRUMENT MIT KNOCHENSCHRAUBE**
SURGICAL INSTRUMENT WITH BONE SCREW
DISPOSITIF CHIRURGICAL AVEC VIS D'OSTÉOSYNTHÈSE

(30) Priorität: 08.06.2009 DE 102009024554; 05.05.2010 DE 102010016812
(43) Veröffentlichungstag der Anmeldung: 18.04.2012
(73) Patentinhaber: Z-Medical GmbH&Co. Kg, 78532 Tuttlingen (DE)
(72) Erfinder: COMBROWSKI, Zbigniew, 78532 Tuttlingen (DE)
(74) Vertreter: Arat, Dogan
(86) Internationale Anmeldenummer: PCT/EP2010/003421
(87) Internationale Veröffentlichungsnummer: WO 2010/142414

(56) Entgegenhaltungen:
- WO-A1-2004/072496
- DE-A1- 19 831 336
- FR-A1- 2 820 630
- US-A1- 2003 040 751
- US-A1- 2007 218 750

## Beschreibung

Die vorliegende Erfindung betrifft ein Chirurgieinstrument nach dem Oberbegriff des Anspruchs 1.

### Stand der Technik

Aus dem Stand der Technik ist bekannt, Knochenschrauben einstückig mit einem Hilfsmittel zum Einbringen der Knochenschraube in einen Knochen zu verbinden. Bei Überschreitung eines bestimmten Drehmoments schert das Hilfsmittel an einer Sollbruchstelle von der Knochenschraube ab.

So zeigt beispielsweise die US 6,732,099 B1 eine Knochenschraube zur Fixierung von Knochen, die mit einem Griff, ähnlich dem eines Schraubendrehers, über eine Sollbruchstelle verbunden ist. Bei Überschreitung eines bestimmten Drehmoments schert der Griff an der Sollbruchstelle ab. Allerdings ist dieser Griff sehr dünn, kurz und massiv ausgeführt. Das hat den Nachteil, dass die Kraft zum Abscheren relativ klein bzw. nicht genau definiert, ist, da sie während des Bruchs abnimmt.

Zudem liegt die Abscherstelle etwas erhöht und stellt eine Störstelle dar, die zu Gewebeirritationen führen kann. Auch besteht bei dieser Knochenschraube die Gefahr, dass sie bei nicht gleichmässig aufgebrachter Kraft oder einem Winkelfehler beim Eindrehen vorzeitig abschert. Ein weiteres Eindrehen ist dann nur erschwert möglich. Eventuell muss die Schraube sogar aufwendig und mit Schaden für den Patienten verbunden wieder entfernt werden.

Ein Chirurgieinstrument nach dem Oberbegriff des Anspruchs 1 ist aus WO 2004/072496 bekannt.

### Aufgabe

Aufgabe der vorliegenden Erfindung ist es, ein Chirurgieinstrument zu schaffen, das auf einfache Art und Weise eingebracht werden kann und bei der die Abscherkraft möglichst definiert ist, weniger Kraft zum Einschrauben benötigt wird, Haltefehler beim Einschrauben nicht sofort zu einem vorzeitigen Abbrechen führen und die Abscherstelle den Heilungsprozess möglichst wenig beeinträchtigt. Außerdem soll möglichst kein zusätzlicher Aufwand mit Sterilisierung entstehen und trotzdem höchsten Anforderungen der Sterilität Rechnung getragen werden. Desweiteren soll die Prozesssicherheit der OP dahingehend verbessert werden, dass die Möglichkeit des Verlierens der Knochenschraube im Körper bis zum Einsetzen in den Knochen vermieden wird. Des weiteren soll erreicht werden, dass nach dem Abscheren des Schafts von dem im Knochen verbleibenden Schraubenteil, ein weiteres Eindrehen des im Knochen verbleibenden Schraubenteils möglich ist.

### Lösung der Aufgabe

Zur Lösung der vorliegenden Aufgabe führt der kennzeichnende Teil des Anspruchs 1.

Ein erfindungsgemäßes Chirurgieinstrument weist in einem bevorzugten Ausführungsbeispiel eine Knochenschraube mit einem Schraubenkopf, und einen Schaft zum Eindrehen der Knochenschraube in einen Knochen auf.

Ein erfindungsgemässes Chirugieinstrument weist in einem bevorzugten Ausführungsbeispiel einen Schaft auf, der als Griff ausgeformt ist. Vorteilhaft hierbei ist dann, dass die Handhabung des Chirurgieinstruments vereinfacht wird.

Ein erfindungsgemässes Chirurgieinstrument mit Schaft und / oder Griff weist in einem bevorzugten Ausführungsbeispiel einen mehrteiligen Griff auf, wobei dieser bevorzugt in Längsrichtung ausziehbar ist, damit der Operateur jederzeit alle Schraubenimplantate einzeln in die gewünschte Position ohne zusätzliche Instrumente eindrehen kann.

Die Knochenschraube des Chirurgieinstruments kann in einem Ausführungsbeispiel ein ein- oder mehrgängiges Gewinde aufweisen. Dies bietet den Vorteil eines möglichst breiten Anwendungsgebietes einer solchen Knochenschraube.

In einem anderen Ausführungsbeispiel kann die Knochenschraube kanuliert ausgeführt sein, wobei diese Kanulierung auch Querbohrungen von einem im Innern der Knochenschraube verlaufenden Kanal umfasst. Diese Kanulierung in Verbindung mit den Querbohrung bietet den Vorteil, dass medizinisch wirkende Stoffe über den Schaft und anschließend durch den Schraubenkopf und die dort bestehende Kanulierung und die Querbohrungen in der Knochenschraube direkt in den Knochen verbracht werden können. Außerdem kann auf gleichem Wege auch eine Füllmasse zur Stützung des Knochens und der besseren Verbindung in den Knochen verbracht werden. Als Füllmasse kommt speziell für diesen Prozess hergestellter z.B. flüssiger Zement oder Gips in Betracht. Diese Schrauben sind sowohl für offene OP's, als auch für minimal invasive Eingriffe einsetzbar.

In einem bevorzugten Ausführungsbeispiel ist der Schaft und die Knochenschraube einstückig ausgebildet. Dies hat den Vorteil, dass ein steriles Chirurgieinstrument direkt an den Nutzer geliefert wird, welcher nach dem Eindrehen der Knochenschraube den Schaft entsorgen kann.

Ein erfindungsgemäßes Ausführungsbeispiel weist zwischen dem Schaft und dem Schraubenkopf der Knochenschraube eine Sollbruchstelle auf. Diese Sollbruchstelle ist geeignet bei Abknicken des Schafts oder Erreichen eines definierten Drehmoments zu brechen. Die benötigte Kraft / Drehmoment zum Abknicken des Schaftes soll den Bedürfnissen an die Knochenschraube im Krankenhaus angepasst sein. Ein Nachdrehen der Schraube nach dem Abbrechen des Schafts und / oder Griffs ist über geeignete Mitnehmer außen oder innen im Schraubenkopf oder innerhalb der Kanulierung jederzeit möglich.

In einem anderen bevorzugten Ausführungsbeispiel eines Chirurgieinstrument ist die Sollbruchstelle in einer ringförmig versenkten Kerbe / Sollbruchstelle des Schraubenkopfes angeordnet ist. Die Tiefe der Kerbe / Sollbruchstelle ist bevorzugt zwischen 0,01 mm und 10 mm, noch bevorzugter ist eine Tiefe zwischen 0,1 mm und 10 mm. Die Tiefe der Kerbe / Sollbruchstelle ist ab dem höchsten Punkt des Schraubenkopfs zu ermitteln. Vorteilhaft an der Kerbe / Sollbruchstelle ist der Umstand, dass die benötigte Kraft zum Abknicken / Abdrehen des Schafts vom Schraubenkopf verringert wird und im Umkehrschluss die Sollbruchstelle in der Weise stabil ausgeführt sein kann, dass der Nutzer beim Eindrehen der Knochenschraube trotzdem genügend Kraft aufbringen kann, um die Knochenschraube in die Knochenmasse zu drehen. Denkbar ist auch, die ringförmige Sollbruchstelle für höhere Drehmomente stabiler auszulegen, und Sollbruchstelle mit Hilfe z.B. einer speziellen Zange anzubrechen oder abzuscheren.

Das erfindungsgemäße Chirurgieinstrument weist als Hilfsmittel zum Einbringen einen Schaft und einer später im Knochen verbleibenden Knochenschraube mit einem zweckmäßigerweise selbstschneidenden Gewinde auf. Dabei weisen Schaft und Knochenschraube zumindest bereichsweise eine Kontur auf, die so ausgebildet ist, dass sie mit dem erfindungsgemäßen Chirurgieinstrument aufgenommen oder in Wirkverbindung bringbar ist. D.h. nach dem Abscheren des Schafts von dem im Knochen verbleibenden Schraubenteil kann der erfindungsgemässe Schaft zweckmässigerweise ohne Wechsel seiner Aufnahme oder anderer Einstellmaßnahmen an der Knochenschraube angesetzt werden und diese noch weiter in den Knochen eindrehen.

Ebenso kann das erfindungsgemäße Chirurgieinstrument zur Reimplantation der Knochenschraube dienen. Dazu wird er an einer dafür geeigneten Kontur am Schraubenteil angesetzt und entgegen der Eindrehrichtung gedreht.

Das erfindungsgemäße Ausführungsbeispiel weist in dem Schraubenkopf einen Mitnehmer auf. Dieser Mitnehmer kann verschiedene Formen aufweisen. Eine besonders bevorzugte Mitnehmer stellt sich als Innenvier- oder Innensechskant dar. Vorteilhaft hierbei ist, dass der Nutzer durch ein dem Innenvielzahn entsprechendes Gegenstück als Ausformung des Schafts zunächst die Knochenschraube in den Knochen eindrehen kann und den Schaft anschließend entfernt. Vorteilhaft ist hierbei auch, dass der Nutzer durch späteres Wiederaufsetzen des Schafts die Knochenschraube weiter in den Knochen eindrehen oder aus dem Knochen herausdrehen kann.

Das erfindungsgemäße Ausführungsbeispiel weist einen Schaft auf, welcher eine Bohrung in Längsrichtung aufweist, durch die im Schraubenkopf ein innenliegender Mitnehmer mit dem Innenvielzahn einbringbar ist. Vorteilhaft ist hierbei, dass eine möglichst verlustfreie und kraftschlüssige Kraftübertragung erreicht wird.

Bei einem anderen bevorzugten Ausführungsbeispiel weist der Mitnehmer im Schraubenkopf einen Freistich oder eine zusätzliche Senkung auf. Dieser Freistich oder die zusätzliche Senkung bietet den Vorteil, dass der Mitnehmer ohne bleibende Grate, Späne gefertigt werden kann, und somit keine Toträume entstehen, die nicht mehr sterilisierbar sind.

Ein weiteres bevorzugtes Ausführungsbeispiel weist ein Verbindungsstück auf, welches als Teil des Schafts ausgebildet ist. Dieses Verbindungsstück soll geeignet sein mit einem Knauf oder einem Stangenelement zusammenzuwirken. Dies kann in Form eines Stecksystems ausgeführt werden. Wichtig ist in diesem Zusammenhang, dass der Knauf in der Weise mit dem Schaft verbunden wird, als dass der Nutzer den Schaft und damit die Knochenschraube betätigen kann. Anschließend soll der Nutzer den Knauf einzeln abnehmen können oder gemeinsam mit dem Schaft von der Knochenschraube abknicken können.

Ein weiteres Ausführungsbeispiel des Knaufs nach ist mehrteilig aufgebaut. Dies kann bedeuten, dass der Knauf sich aus verschiedenen trennbaren Teilen besteht. Es kann aber auch bedeuten, dass der Knauf aus ineinander versenkten Teilen besteht die wie ein Teleskop auseinandergezogen werden können, wobei sich die Gesamtlänge des Knaufs dadurch verändern lässt. Vorteilhaft ist hierbei ein möglichst vielseitiger Einsatz des Knaufs.

In einem bevorzugten Ausführungsbeispiel sind Schaft und Knauf als Einwegartikel ausgelegt und sollen nach dem Eindrehen der Knochenschraube entsorgt werden können. Dies bietet den Vorteil einer sterilen Nutzung und anschließenden einfachen Entsorgung.

Die Knochenschraube und der Schaft werden einstückig und steril geliefert. Der Nutzer kann dann den Knauf aufsetzen und mithilfe des Schafts die Knochenschraube in den Knochen drehen. Der Knauf kann auch als Mehrwegartikel ausgelegt sein. Vorteilhaft hierbei ist der Umstand, dass keine zusätzliche Sterilisierung von entsprechenden Gerätschaften aus dem Stand der Technik mehr notwendig ist.

Die Knochenschraube kann kanuliert oder nicht kanuliert ausgebildet sein. Ebenso kann auch der Schaft und / oder der Knauf kanuliert oder nur hohl ausgebildet sein. Der Unterschied besteht darin, dass bei einer Kanulierung ein Kanal vom oberen Ende des Schafts, also der Stelle welche über das Verbindungselement mit dem Knauf verfügt, und dem unteren Ende des Schafts, also welcher an die Knochenschraube anschließt. Bei einem hohlen Schaft und / oder der Knauf kann der obere oder der untere Bereich auch geschlossen ausgebildet sein.

In einem bevorzugten Ausführungsbeispiel besitzt die Knochenschraube eine selbstbohrende Spitze und / oder ein selbstschneidendes Gewinde. Vorteilhaft ist hierbei, dass die Knochenschraube dadurch möglichst einfach in den Knochen eingedreht werden kann und der Kraftaufwand des Nutzers dadurch verringert wird.

Der Schaft zur Implantation und der im Knochen verbleibende Schraubenteil der Knochenschraube sind einstückig über eine Sollbruchstelle miteinander verbunden. Vorzugsweise ist in den Schaft eine Bohrung eingebracht, so dass der Schaft und der Schraubenkopf nur über eine ringförmig ausgebildete Fläche bzw. eine Abscherfläche, verbunden sind.

Mit dem Begriff "ringförmig" werden in der vorliegenden Anmeldung nicht nur runde Formen beschrieben, sondern alle Formen, die in einem Innenbereich eine Aussparung oder einen Ausschnitt aufweisen. Auch eckige, ovale oder unregelmäßige Rahmen fallen unter die Bezeichnung "ringförmig". Beispielhaft sollen hier ein Rechteck mit einem rechteckigen Ausschnitt oder ein Oval mit einem runden Ausschnitt genannt sein. Vom Erfindungsgedanken ist auch umfasst, dass eine ringförmige Verbindungsfläche, d.h. die Sollbruchstelle, perforiert ausgebildet ist.

Die Bohrung im Schaft ermöglicht zum einen, einen Innenvielkant im Schraubenkopf einzubringen, zugleich ergibt sich durch die ringförmige Ausbildung der Sollbruchstelle ein definierter Hebelarm über den Radius, und somit ein definiertes Moment zum Abreißen des Schafts von der Knochenschraube bei hoher Seitenstabilität. Ein weiterer Vorteil der Bohrung im Schaft ist, dass eine falsche Haltung der Knochenschraube bei der Implantation, nicht so leicht, wie bei vergleichbaren Schrauben zu einem vorzeitigen Abscheren führt, da über den Radius der Bohrung eine höhere Seitenkraft erreichbar ist.

Die Sollbruchstelle liegt zweckmässigerweise versenkt in einem Schraubenkopf des im Knochen verbleibenden Schraubenteils. Das hat den Vorteil, dass auch die spätere Abscherfläche versenkt im Schraubenkopf liegt und mit ihrer rauen Oberfläche nicht zu Irritationen in einem umliegenden Gewebe, wie Knochen, Haut oder Fleisch führen kann.

Das Gewinde der Knochenschraube ist vorzugsweise konisch ausgeführt. Dadurch kann die Knochenschraube leichter im- und reimplantiert werden. Es wird weniger Kraft zum Eindrehen bzw. zum Schneiden des Gewindes bei der Implantation benötigt. Ein weiterer Vorteil eines konischen Gewindes ist, dass sich eine bessere Kompression ergibt. Ebenfalls vom Erfindungsgedanken umfasst, sind mehrgängig ausgeführte Gewinde.

Ein bevorzugtes Ausführungsbeispiel für das Stangenelement ist ein zylindrischer Körper, welcher entweder als Hohlrohr oder als Vollkunstoffkörper oder aber als zylindrischer Körper mit einer Kanulierung ausgeformt ist. Vorteilhaft hierbei ist, dass das Stangenelement aufgrund seiner länglichen Form die Arbeit des Nutzers erleichtert, da die Knochenschraube ein- oder ausgedreht werden kann, ohne dass der Nutzer hierzu in das den Knochen umgebende Gewebe hineingreifen muss.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Figur 1: eine Draufsicht auf ein Chirurgieinstrument;
- Figur 2: einen Schnitt entlang einer Linie II-II in Figur 1;
- Figur 3: eine perspektivische Darstellung des Chirurgieinstrumentes gemäss Figur 1;
- Figur 4: eine Seitenansicht eines Schraubendrehers mit einer Knochenschraube;
- Figur 5: eine Draufsicht auf den Schraubendreher gemäss Figur 4 um 90° gedreht;
- Figur 6: eine perspektivische Darstellung einer Halterung für einen Schraubendreher;
- Figur 7: eine perspektivische Ansicht eines Schraubendrehers mit der Halterung gemäß Figur 6;
- Figur 8: einen vergrösserten Ausschnitt aus dem Schraubendreher gemäß Figur 4 im Ansatzbereich an einer Knochenschraube;
- Figur 9: eine Vorderansicht eines Schraubendrehers mit einer Knochenschraube und einem Magazin
- Figur 10: eine geschnittene Seitenansicht eines Chirurigieinstruments und eines Knaufs;
- Figur 11: eine vergrösserte Seitenansicht eines Ausschnitts aus Figur 10 von seitlich oben;
- Figur 12: eine geschnittene Seitenansicht eines anderen Ausführungsbeispiels eines getrennten Chirurgieinstruments;
- Figur 13: eine vergrößerte Ansicht von schräg oben eines Teils eines zusammengesetzten Chirurgieinstruments nach Figur 12;
- Figur 14: eine geschnittene Seitenansicht eines Chirurgieinstruments nach Figur 10 mit aufgesetztem Knauf;
- Figur 15: eine vergrößerte geschnittene Seitenansicht eines Teils des erfindungsgemässen Chirurgieinstruments nach Figur 14.

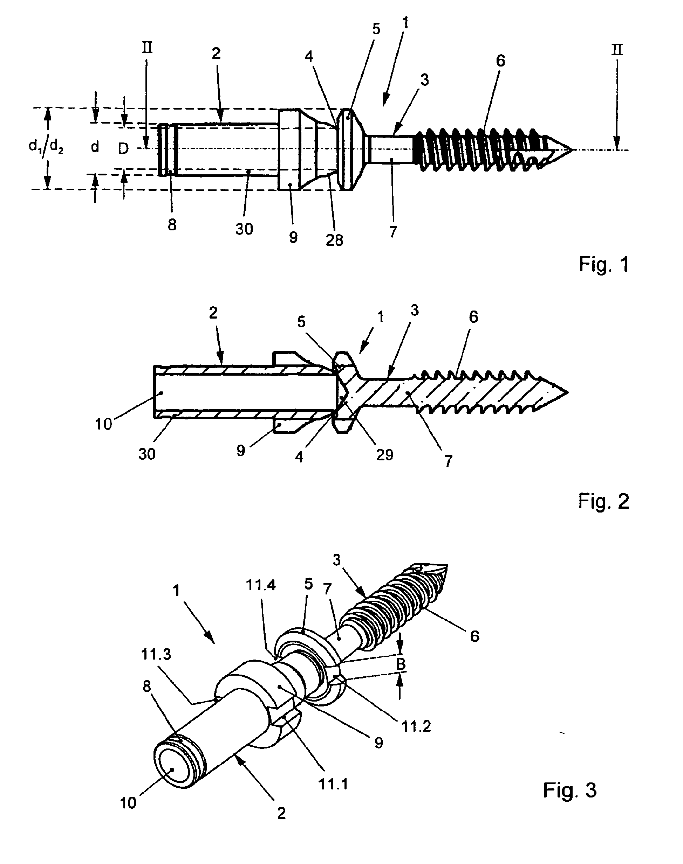

Figur 1 zeigt eine Knochenschraube 1. Diese weist einen Schaft 2 als Hilfsmittel zum Einbringen und einen Schraubenteil 3 auf. Der Schraubenteil 3 selbst ist aus einem Schraubenkopf 5 und einem Schraubenschaft 7 gebildet. Der Schraubenschaft 7 des Schraubenteils 3 ist über ca. 2/3 seiner Länge mit einem selbstschneidenden Gewinde 6 versehen.

Der Schaft 2 ist über eine Sollbruchstelle 4 einstückig am Schraubenkopf 5 mit dem Schraubenteil 3 verbunden. Am Umfang eines Hülsenabschnitts 30 weist der Schaft 2 eine umlaufende Nut 8 und an den Hülsenabschnitt 30 angeformt einen Vorsprung 9 auf. Der Vorsprung 9 verjüngt sich an einem Ende 28 auf einen Durchmesser D, kleiner als ein Durchmesser d des Schaftes 2 und ist mit diesem verjüngten Ende 28 einstückig mit dem Schraubenteil 3 bzw. dem Schraubenkopf 5 verbunden. Das verjüngte Ende 28 liegt etwas versenkt in einer Mulde 29 im Schraubenkopf 5 und stellt die Sollbruchstelle 4 dar.

Wie der Schnitt in Figur 2 zeigt, weist der Schaft 2 eine zentrische Bohrung 10 auf. Diese Bohrung 10 durchzieht den Schaft 2, so dass eine Abscherfläche an der Sollbruchstelle 4 ringförmig ausgebildet ist.

Der Schraubenkopf 5 weist eine ähnliche Aussenkontur wie der Absatz 9 auf. Der Durchmesser d₁ des Schraubenkopfes 5 entspricht dem Durchmesser d₂ des Absatzes 9. Auch sind in den Schraubenkopf 5 und in den Absatz 9 jeweils zwei rechteckige Kerben 11.1 und 11.3 sowie 11.2 und 11.4 mit einer Breite B eingebracht. Wie in Figur 3 gezeigt, fluchten jeweils die Kerben 11.1 und 11.2 wie auch die gegenüberliegenden Kerben 11.3 und 11.4.

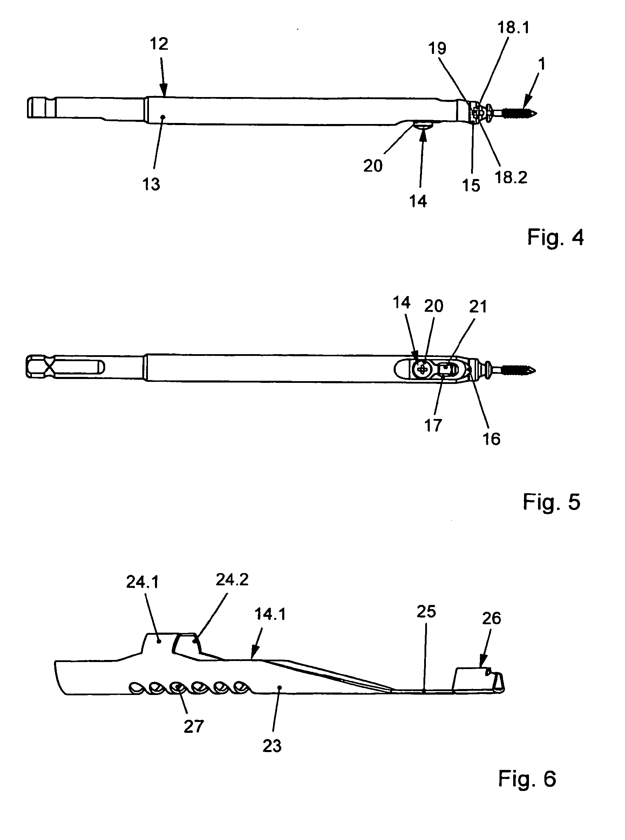

Weiter zeigt Figur 4 einen Schraubendreher 12 zur Implantation einer Knochenschraube 1. Der Schraubendreher 12 weist einen rohrförmigen Grundkörper 13, eine Halterung 14 und eine Schaft- und Schraubenaufnahme 15 auf.

In ihrem vorderen Bereich ist die Schaft- und Schraubenaufnahme 15 aus Hohlmaterial gebildet. In die Schaft- und Schraubenaufnahme 15 sind zwei axiale, rechteckförmige Ausschnitte 18.1 und 18.2 so eingebracht, dass zwischen ihnen eine Nase 19 ausgebildet wird. Nicht dargestellt, sind gegenüberliegend ebenfalls solche Ausschnitte und eine entsprechende Nase angeordnet. Dabei ist die Nase 19 schmäler als die Breite B der Kerben 11.1 bis 11.4 ausgeführt, so dass die Nasen 19 jeweils in die gegenüberliegende Kerben 11.1 und 11.3 im Schaft 2 oder die Kerben 11.2 und 11.4 im Schraubenkopf 5 greifen können.

Für die Halterung 14 ist eine Ausnehmung 16 in den rohrförmigen Grundkörper 13, der als Vollmaterial ausgebildet ist und in die Schaft- und Schraubenaufnahme 15, die als Hohlmaterial ausgebildet ist, eingebracht. Im Hohlmaterial der Schrauben- und Schaftaufnahme 15 ist zudem ein Durchbruch 17 vorgesehen. In das Vollmaterial des Grundkörpers 13 ist mittels einer Befestigungsschraube 20 ein Halteelement 21 befestigt. Das Halteelement 21 ragt in die Ausnehmung 16 und durch den Durchbruch 17 in einen Innenraum der Schaft- und Schraubenaufnahme 15. Dabei ist das Halteelement 21 so gebogen, dass es in die Nut 8 im Schaft 2 der Knochenschraube 1 eingreifen kann. So wird die Knochenschraube 1 im Schraubendreher 12 gehalten.

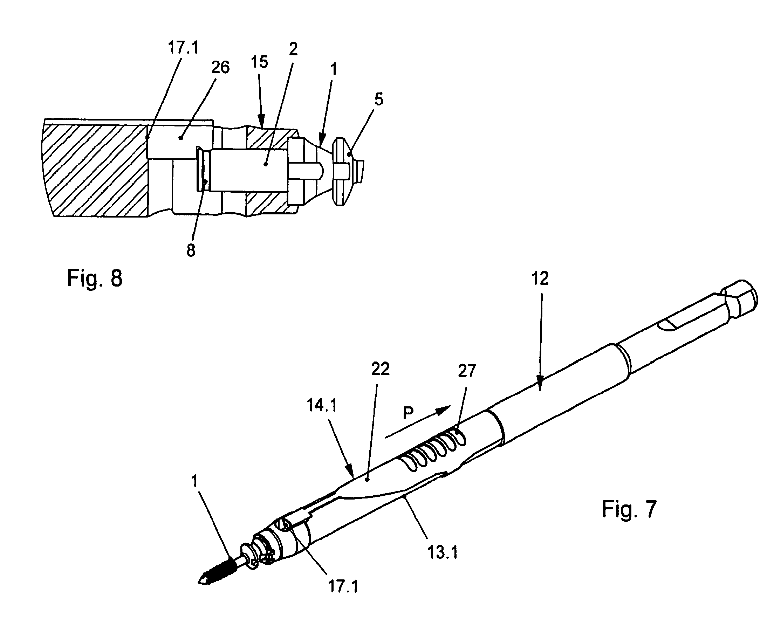

Die Figur 7 zeigt ein weiteres, besonders bevorzugtes Ausführungsbeispiel eines Schraubendrehers 12.1. Der Schraubendreher 12.1 entspricht im Wesentlichen dem Schraubendreher 12, unterscheidet sich aber in der Ausbildung einer Halterung 14.1.

Die Halterung 14.1 ist einstückig aus einem Federelement, siehe Figur 6, gebildet und weist einen schalenartigen Grundkörper 23 mit Haltelaschen 24.1 und 24.2 auf. In den Grundkörper 23 sind, um eine bessere Griffigkeit zu gewährleisten, die Flexibilität zu erhöhen und um Gewicht und Material zu sparen, Schlitze 27 eingebracht.

Der Grundkörper 23 mündet in einen schmalen Steg 25, der an seiner Spitze eine Art Haken 26 ausbildet. Die Halterung 14.1 wird mit dem schalenartigen Grundkörper 23 und den Haltelaschen 24.1 und 24.2 um einen rohrförmigen Grundkörper 13.1 des Schraubendrehers 12.1 gelegt. Dabei greift der Haken 26, wie aus dem Schnitt in Figur 8 deutlich zu entnehmen ist, durch einen Durchbruch 17.1 und in die Nut 8 in der Knochenschraube 1.

Die Funktionsweise der vorliegenden Knochenschraube ist folgende:

Zur Implantation der Knochenschraube 1 wird diese in einen Schraubendreher 12 oder 12.1 gesetzt. Dabei greifen die Nasen 18.1 bzw. 18.2 in die Kerben 11.1 und 11.3 im Absatz 9 des Schaftes 2 der Knochenschraube 1 ein.

Um zu verhindern, dass die Knochenschraube 1 aus dem Schraubendreher 12 oder 12.1 herausfällt, wird sie über die Halterung 14 bzw. 14.1 durch Eingriff des Hakens 26 in ihrer Nut 8 gehalten. Beim Schraubendreher 12.1 wird zur Aufnahme der Knochenschraube 1 das Federelement in Pfeilrichtung P zurückgezogen, dann kann die Knochenschraube 1 eingesetzt werden und das Federelement wird entgegen der Pfeilrichtung P geschoben so, dass der Haken 26 in die Nut 8 greift und die Knochenschraube 1 hält.

Jetzt kann die Knochenschraube 1 mit dem Schraubendreher 12 oder 12.1 in einen Knochen bzw. in eine bereits vorgeschnittene Bohrung implantiert bzw. eingedreht bzw. eingeschraubt werden. Dabei schneidet sich die Knochenschraube 1 mit ihrem Gewinde 6 selbst einen Gewindegang in das Knochengewebe.

Bei Überschreitung eines definierten Drehmoments schert der Schaft 2 an der Sollbruchstelle 4 vom Schraubenkopf 5 und dem Schraubenteil 3 ab. Dadurch bildet sich eine nicht dargestellte, ringförmige Abscherftäche. Diese liegt, wie aus Figur 3 erkennbar wird, leicht versenkt im Schraubenkopf 5. Das hat den Vorteil, dass die Abscherfläche, die meist rau ausgebildet ist, nicht zu Irritationen in der Haut oder im Fleisch führen kann.

Beim Schraubendreher 12.1 ist besonders vorteilhaft, dass durch Schieben des Halteelementes 14.1 in Gegenpfeilrichtung P der abgescherte Schaft 2 durch den Haken 26 nach vorne aus dem Schraubendreher 12.1 bzw. dessen rohrförmigen Grundkörper 13.1 ausgestossen werden kann.

Nach Entfernen des Schaftes 2 kann der Schraubenteil 3 der Knochenschraube 1 mit dem Schraubendreher 12 oder 12.1 noch weiter in die vorgeschnittene Bohrung eingebracht werden. Dazu wird der Schraubendreher 12 bzw. 12.1 mit seinen Nasen 19 in die Kerben 11.2 und 11.4 am Schraubenkopf 5 eingesetzt. Genau so kann das Schraubenteil 3 durch Ändern der Drehrichtung auch wieder aus dem Knochen entfernt werden.

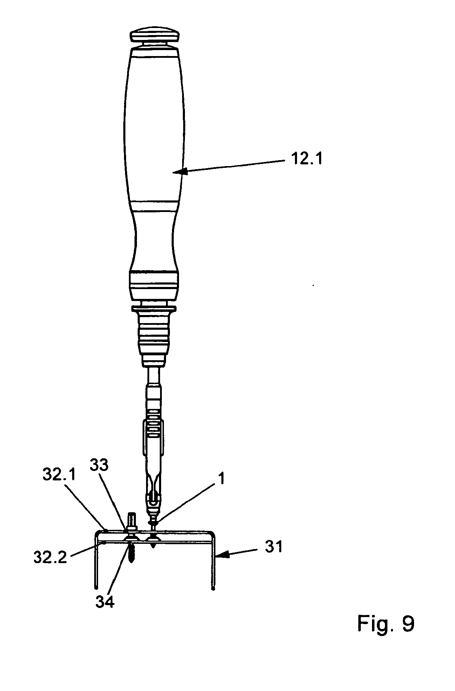

Zweckmäßigerweise wird ein Bausatz aus einem Schraubendreher 12 bzw. 12.1 und einer Knochenschraube 1 durch ein in Figur 9 dargestelltes Magazin 31 ergänzt. Dieses Magazin 31 dient dazu, Knochenschrauben 1 verdrehsicher aufzunehmen und zur Entnahme mit dem Schraubendreher 12 bzw. 12.1 bereitzustellen. Um diese Aufgabe zu erfüllen, weist es zwei Halteplatten 32.1 und 32.2 auf. Diese sind übereinander in einem Abstand, der in etwa einer Höhe von Schraubenkopf 5 und Absatz 9 entspricht, angeordnet und weisen fluchtende Öffnungen 33 und 34 auf. Die Öffnung 33 ist grösser als der größte Durchmesser d₁ der Knochenschraube 1 ausgebildet. Die Knochenschraube kann also durch die Bohrung geführt werden. Dabei ist die Öffnung 34 so aufgeformt, dass die Knochenschraube 1 mit ihrem Schraubenkopf 5 an der unteren Halteplatte 32.2 hängen bleibt. Vorzugsweise dient ein solches Magazin zur Aufnahme von mehreren Knochenschrauben, auch in verschiedenen Längen.

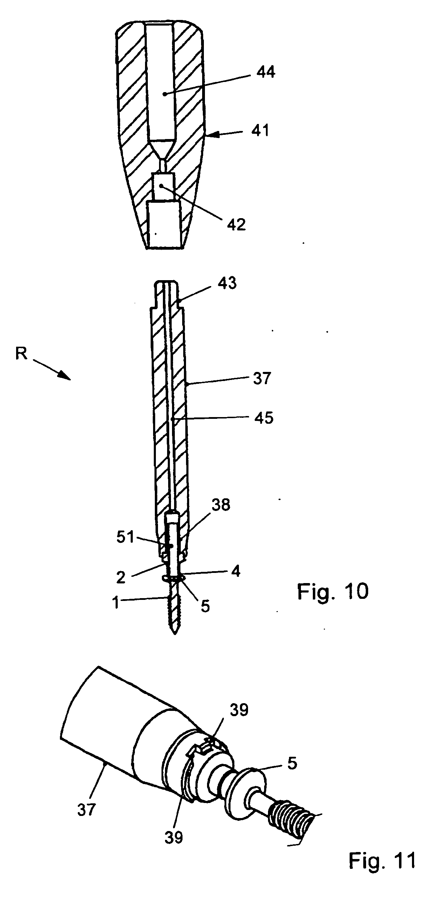

In Figur 10 ist ein weiteres Chirurgieinstrument R gezeigt. Diese besteht aus dem Schaft 2 und der Knochenschraube 1. An der Knochenschraube 1 ist der Schraubenkopf 5 angeformt. Der Schaft 2 wirkt hier mit einem Stangenelement 37 zusammen. Vorteilhaft hierbei ist, dass Transport- und Verpackungskosten gespart werden, wenn der Schaft 2 in der Form verkleinert wird, dass nur noch die Knochenschraube 1 gemeinsam mit dem Schaft 2 geliefert wird.

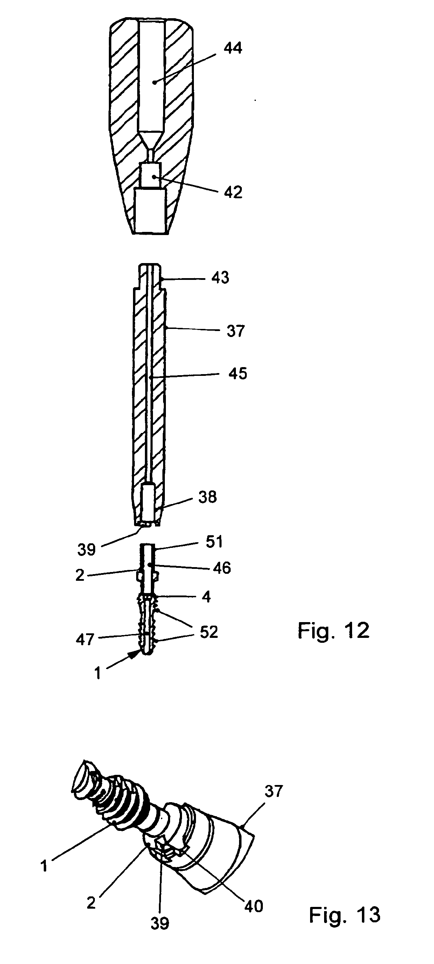

In diesem Chirurgieinstrument wird der Schaft 2 zur Zentrierung in eine Aufnahme 38 des Stangenelements 37 eingebracht. Ausserdem verfügt das Stangenelement 37 außerdem über Einkerbungen 39, welche mit entsprechenden Eingriffsnasen 40, wie sie in Figur 13 zu erkennen sind, zusammenwirken und das Eindrehen der Knochenschraube 1 über den Schaft 2 bewirken.

Ausserdem ist in Figur 10 ein Knauf 41 gezeigt. Dieser Knauf 41 verfügt über eine Aufnahme 42, welche der Aufnahme des Stangenelements 37, insbesondere dem Verbindungselement 43 dient. Ausserdem ist in Figur 10 gut zu erkennen, wie eine durchgehende Kanulierung durch alle gezeigten Teile erreicht wird. Diese durchgehende Kanulierung beginnt mit Kanal 44 in dem Knauf 41, setzt sich fort in einem Kanal 45 des Stangenelements 37. Dass diese Kanulierung sich bis zu der Knochenschraube 1 fortsetzen kann ist in Figur 12 gut anhand des Kanals 46 im Schaft 2 und einem Kanal 47 in der Knochenschraube zu erkennen.

Figur 12 zeigt eine Knochenschraube 1, bei der der Schraubenkopf 5 ohne Wulst ausgebildet ist. Dies hat den Vorteil, dass die Knochenschraube 1 in einem nicht gezeigten Knochen vollständig versenkt werden kann. Ausserdem ist gut zu erkennen, wie die Knochenschraube 1 in Figur 12 ein mehrgängiges Gewinde 48 aufweist.

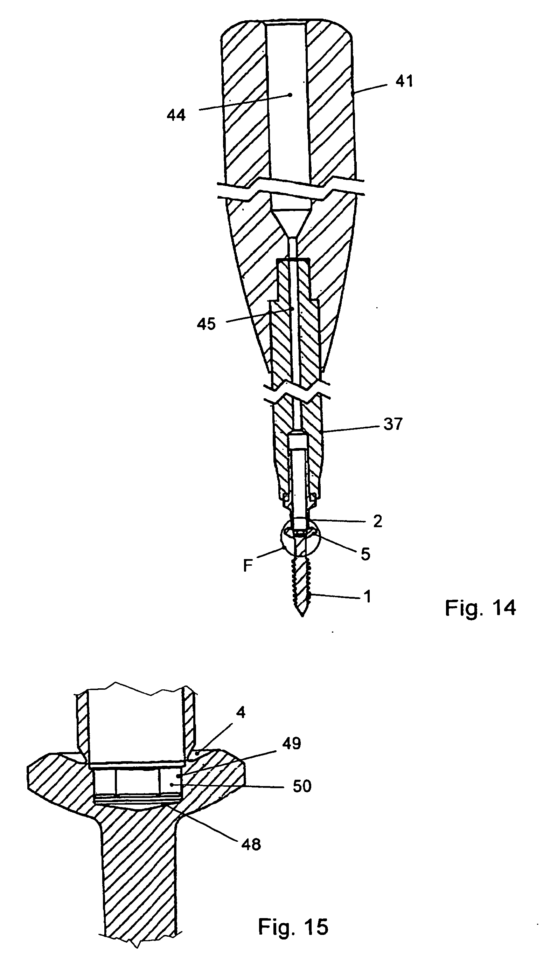

Figur 14 zeigt das Chirurgieinstrument R, welches aus dem Schaft 2 und der Knochenschraube 1 besteht, welche im Bereich des Schraubenkopfs 5 einstückig miteinander verbunden sind. Der Bereich F ist in Figur 15 nochmals vergrößert dargestellt.

In Figur 15 ist nun gut zu erkennen wie die Sollbruchstelle 4 in Form einer Mulde ringförmig ausgebildet ist. Ausserdem ist ein Innenvielzahn 50 sowie ein Mitnehmer 49 gezeigt, welche kraftschlüssig ineinander greifen. Dabei ist der Mitnehmer als Teil der Knochenschraube 1 und der Innenvielzahn 50 als Teil des Schafts 2 ausgeformt. Ausserdem ist ein Freistich oder zusätzliche Senkung 48 zu erkennen.

### Bezugszeichenliste

| | | | | | |
|---|---|---|---|---|---|
| 1 | Knochenschraube | 34 | Öffnung | 67 | |
| 2 | Schaft | 35 | | 68 | |
| 3 | Schraubenteil | 36 | | 69 | |
| 4 | Sollbruchstelle | 37 | Stangenelement | 70 | |
| 5 | Schraubenkopf | 38 | Aufnahme | 71 | |
| 6 | Gewinde | 39 | Kerbe | 72 | |
| 7 | Schraubenschaft | 40 | Eingriffsnase | 73 | |
| 8 | Nut | 41 | Knauf | 74 | |
| 9 | Absatz | 42 | Aufnahme | 75 | |
| 10 | Bohrung | 43 | Verbindungselement | 76 | |
| 11 | Kerbe | 44 | Kanal | 77 | |
| 12 | Schraubendreher | 45 | Kanal | 78 | |
| 13 | Grundkörper | 46 | Kanal | 79 | |
| 14 | Halterung | 47 | Kanal | | |
| 15 | Schaft-und Schraubenaufnahme | 48 | Senkung | | |
| 16 | Öffnung | 49 | Mitnehmer | D | Durchmesser |
| 17 | Durchbruch | 50 | Innenvielzahn | d | Durchmesser von 2 |
| 18 | Ausschnitt | 51 | Verbindungsstück | B | Breite |
| 19 | Nase | 52 | Gewinde | P | Pfeilrichtung |
| 20 | Befestigungsschraube | 53 | | d₁ | Durchmesser von 5 |
| 21 | Halteelement | 54 | | d₂ | Durchmesser von 9 |
| 22 | | 55 | | | |
| 23 | Grundkörper | 56 | | | |
| 24 | Haltelasche | 57 | | | |
| 25 | Steg | 58 | | | |
| 26 | Haken | 59 | | | |
| 27 | Schlitz | 60 | | | |
| 28 | verjünqtes Ende | 61 | | | |
| 29 | Mulde | 62 | | | |
| 30 | Hülsenabschnitt | 63 | | | |
| 31 | Magazin | 64 | | | |
| 32 | Halteplatte | 65 | | | |
| 33 | Öffnung | 66 | | | |

## Patentansprüche

1. Chirurgieinstrument mit
einer Knochenschraube (1) mit einem Schraubenkopf (5) und
einem Schaft (2) zum Eindrehen der Knochenschraube (1) in einen Knochen, wobei der Schaft (2) mit der Knochenschraube (1) einstückig ausgebildet ist, wobei der Schaft (2) als Griff ausgeformt ist und zwischen dem Schaft (2) und dem Schraubenkopf (5) der Knochenschraube (1) eine Sollbruchstelle (4) vorgesehen ist,
**dadurch gekennzeichnet,**
**dass** der Schraubenkopf (5) einen Mitnehmer (49) und der Schaft (2) einen Innenvielzahn (50) aufweist und der Schaft (2) eine Bohrung in Längsrichtung aufweist, durch die der im Schraubenkopf (5) innenliegende Mitnehmer (49) in den Innenvielzahn (50) des Schafts (2) einbringbar ist.

2. Chirurgieinstrument nach Anspruch 1, **dadurch gekennzeichnet, dass** die Sollbruchstelle (4) in einer ringförmig versenkten Mulde des Schraubenkopfes (5) angeordnet ist.

3. Chirurgieinstrument nach einem der vorigen Ansprüche, **dadurch gekennzeichnet, dass** der Mitnehmer (49) im Schraubenkopf (5) eine Senkung (48) aufweist.

4. Chirurgieinstrument nach einem der vorigen Ansprüche, **dadurch gekennzeichnet, dass** der Schaft (2) ein Verbindungsstück (51) aufweist, welches geeignet ist mit einem Knauf (41) oder einem Stangenelement (37) zusammenzuwirken.

5. Chirurgieinstrument nach Anspruch 4, **dadurch gekennzeichnet, dass** das Stangenelement (37) mit dem Knauf (41) zusammenwirkt.

6. Chirurgieinstrument nach den Ansprüchen 1 bis 5, wobei die Knochenschraube ein Gewinde (6, 52) aufweist, welches ein-oder mehrgängig ausgebildet ist.

7. Chirurgieinstrument nach Anspruch 6, **dadurch gekennzeichnet, dass** die Knochenschraube (1) einen im Innern verlaufenden Kanal (47) aufweist, welcher geeignet ist über einen Kanal (46) im Schaft (2) zusammenzuwirken, wobei eine Öffnung im Schraubenkopf (5) vorhanden ist, welcher mit einer anderen Öffnung des Schafts(2) zusammenwirkt.

8. Chirurgieinstrument nach Anspruch 7, **dadurch gekennzeichnet, dass** der Kanal (47) Querbohrungen umfasst, welche vom Kanal (47) aus zu einer Außenfläche verlaufen und geeignet sind einen Wirkstoff oder eine Füllmasse aufzunehmen und zu führen.

## Claims

1. A surgical instrument having
a bone screw (1) with a screw head (5) and
a shaft (2) for screwing of the bone screw (1) into a bone,
wherein the shaft (2) is in one piece with the bone screw (1),
wherein the shaft (2) is formed as a grip and a predetermined breaking point (4) is provided between the shaft (2) and the screw head (5) of the bone screw (1),
**characterised in that**
the screw head (5) has an entrainer (49) and the shaft (2) has an internal serration (50) and the shaft (2) has a bore in the longitudinal direction through which the entrainer (49) lying within the screw head (5) can be introduced into the internal serration (50) of the shaft (2).

2. A surgical instrument according to claim 1, **characterised in that** the predetermined breaking point (4) is arranged in an annularly recessed trough-shaped depression of the screw head (5).

3. A surgical instrument according to any one of the preceding claims, **characterised in that** the entrainer (49) in the screw head (5) has an indentation (48).

4. A surgical instrument according to any one of the preceding claims, **characterised in that** the shaft (2) has a connecting piece (51) suitable for co-operating with a knob (41) or a rod element (37).

5. A surgical instrument according to claim 4, **characterised in that** the rod element (37) cooperates with the knob (41).

6. Surgical instrument according to the claims 1 to 5, wherein the bone screw has a thread (6, 52) which is single-thread or multi-thread.

7. A surgical instrument according to claim 6, **characterised in that** the bone screw (1) has a channel (47) running in the interior, which channel (47) is suitable for co-operating via a channel (46) in the shaft (2), wherein an opening in the screw head (5) is present, which screw head (5) cooperates with another opening of the shaft (2).

8. A surgical instrument according to claim 7, **characterised in that** the channel (47) comprises transverse bores which run out from the channel (47) to an outer surface and are suitable for receiving and guiding an active substance or a filler.

## Revendications

1. Instrument chirurgical, avec une vis d'ostéosynthèse (1) avec une tête de vis (5) et une tige (2) pour introduire la vis d'ostéosynthèse (1) dans un os, la tige (2) étant réalisée d'une seule pièce avec la vis d'ostéosynthèse (1), la tige (2) étant réalisée sous forme de poignée et entre la tige (2) et la tête (5) de la vis d'ostéosynthèse (1) étant prévu un point de rupture de consigne (4),
**caractérisé par le fait**
**que** la tête de vis (5) présente un doigt d'entraînement (49) et la tige (2) présente une denture multiple intérieure (50) et que la tige (2) présente un alésage dans le sens longitudinal à travers lequel le doigt d'entraînement (49) situé dans la tête de vis (5) peut être introduit dans la denture multiple intérieure (50) de la tige (2).

2. Instrument chirurgical selon la revendication 1, **caractérisé par le fait que** le point de rupture de consigne (4) est disposé dans une cavité enfoncée en forme d'anneau de la tête de vis (5).

3. Instrument chirurgical selon l'une des revendications précédentes, **caractérisé par le fait que** le doigt d'entraînement (49) dans la tête de vis (5) présente un enfoncement (48).

4. Instrument chirurgical selon l'une des revendications précédentes, **caractérisé par le fait que** la tige (2) présente un raccord (51) qui convient pour coopérer avec un bouton (41) ou un élément en forme de barre (37).

5. Instrument chirurgical selon la revendication 4, **caractérisé par le fait que** l'élément en forme de barre (37) coopère avec le bouton (41).

6. Instrument chirurgical selon les revendications 1 à 5, dans lequel la vis d'ostéosynthèse présente un filet (6, 52) qui est réalisé à un ou plusieurs pas.

7. Instrument chirurgical selon la revendication 6, **caractérisé par le fait que** la vis d'ostéosynthèse (1) présente un canal (47) s'étendant à l'intérieur qui convient pour coopérer avec un canal (46) dans la tige (2), dans la tête de vis (5) étant présente une ouverture qui coopère avec une autre ouverture de la tige (2).

8. Instrument chirurgical selon la revendication 7, **caractérisé par le fait que** le canal (47) comprend des alésages transversaux qui s'étendent depuis le canal (47) vers une face extérieure et qui conviennent pour recevoir et guider une substance active ou une masse de remplissage.
